(19) Europäisches Patentamt
European Patent Office
Office européen des brevets

(11) **EP 2 499 998 A1**

(12) **EUROPÄISCHE PATENTANMELDUNG**

(43) Veröffentlichungstag:
**19.09.2012 Patentblatt 2012/38**

(51) Int Cl.:
*A61F 2/36* (2006.01)    *A61F 2/32* (2006.01)

(21) Anmeldenummer: **12169546.4**

(22) Anmeldetag: **16.02.2005**

(84) Benannte Vertragsstaaten:
**CH DE FR GB LI**

(30) Priorität: **19.02.2004 DE 102004008138**

(62) Dokumentnummer(n) der früheren Anmeldung(en) nach Art. 76 EPÜ:
**05707439.5 / 1 715 816**

(71) Anmelder: **Mathys AG Bettlach**
**2544 Bettlach (CH)**

(72) Erfinder:
• **Delfosse, Daniel, Dr.**
**3306 Jegensdorf (CH)**

• **Cotting, Anton**
**2540 Grenchen (CH)**
• **Schönholzer, Adrian**
**4542 Luterbach (CH)**

(74) Vertreter: **Körfer, Thomas**
**Mitscherlich & Partner**
**Patent- und Rechtsanwälte**
**Sonnenstrasse 33**
**80331 München (DE)**

Bemerkungen:
Diese Anmeldung ist am 25-05-2012 als Teilanmeldung zu der unter INID-Code 62 erwähnten Anmeldung eingereicht worden.

(54) **Femurkomponente einer Hüftgelenks-Endoprothese mit ovalem Anschlagsbereich**

(57)     Eine Femurkomponente (1) für eine Hüftgelenksprothese umfaßt einen in einer Markhöhle eines Oberschenkelknochens verankerbaren Schaft (2) und eine daran ausgebildeten Halspartie (3) mit einem Zapfen (4) zur Aufnahme eines Gelenkkopfes. Die Halspartie (3) weist in einem zwischen dem Zapfen (4) und dem Schaft (2) ausgebildeten Anschlagsbereich (5) einen nicht runden Querschnitt auf. Der Querschnitt des Anschlagsbereiches (5) ist oval mit zwei verschiedenen Krümmungsradien ($R_1$,$R_2$) und geraden Flanken (16) ausgebildet, wobei gilt:
$R_1 < R_2$,
wobei $R_1$ des Krümmungsradius im medialen Bereich, und $R_2$ der Krümmungsradius im lateralen Bereich ist.

Fig. 1

EP 2 499 998 A1

Fig. 6B

**Beschreibung**

Die Erfindung betrifft eine Femurkomponente für eine Hüftgelenksprothese zur prothetischen Versorgung des menschlichen oder tierischen Hüftgelenks.

Beispielsweise ist aus der WO 98/23231 A1 eine Femurkomponente für eine Hüftgelenks-Endoprothese bekannt. Diese weist einen zur Verankerung in der Markhöhle des Femur bestimmten Schaft auf, an welches sich eine Halspartie mit einem Zapfen zur Aufnahme eines Gelenkkopfes oder einem mit der Halspartie fest verbundenen Gelenkkopf anschließt. Die Halspartie weist dabei einen runden Querschnitt auf.

Ebenso sind ähnliche Hüftgelenks-Endoprothesen aus den Druckschriften WO 83/02555 A1 sowie EP 0 048 745 A1 und EP 0 093 378 A1 bekannt, welche ebenfalls in der Halspartie einen runden Querschnitt mit einer durch den Durchmesser bestimmten Querschnittsfläche aufweisen.

Nachteilig an den aus den oben genannten Druckschriften bekannten Hüftgelenksprothesen ist insbesondere, daß die Querschnittsform der Halspartie rund gestaltet ist und dadurch der sog. Range of motion, welcher den Beweglichkeitsgrad des Oberschenkels relativ zum Becken bezeichnet, eingeschränkt ist. Dies äußert sich in einer Einschränkung der Beweglichkeit des Beins des Patienten und die Gefahr des Anstoßens des metallischen Schaftes an der Hüftpfanne.

Der Erfindung liegt demnach die Aufgabe zugrunde, eine Femurkomponente für eine Hüftgelenks-Endoprothese zu schaffen, welche durch eine geeignete Querschnittsform der Halspartie bei gleicher Querschnittsfläche einen größeren Range of motion erlaubt.

Die Aufgabe wird bezüglich der Hüftgelenksprothese durch die Merkmale der Ansprüche 1 und 11 gelöst.

Gemäß Anspruch 1 ist vorgesehen, die Querschnittsform der Halspartie der Femurkomponente in einem Anschlagsbereich abweichend von einer runden Querschnittsform zu gestalten.

Gemäß Anspruch 11 ist vorgesehen, den Anschlagsbereich durch eine exzentrische Anordnung des Zapfens für den Gelenkkopf zu verkleinern.

Weitere vorteilhafte Ausgestaltungen der Erfindung sind in den Unteransprüchen gekennzeichnet.

Vorteilhafterweise ist der Anschlagbereich im Querschnitt elliptisch, rechteckig oder oval gestaltet, so daß ein Öffnungswinkel zwischen Berührungspunkten der Femurkomponente mit einer Gelenkpfanne vergrößert werden kann.

Weiterhin ist von Vorteil, daß die Vergrößerung des Öffnungswinkels auch durch die exzentrische Verschiebung des den Gelenkkopf tragenden Zapfens relativ zur Halspartie durch Unterschneidung des Anschlagbereiches mit dem Rand der Gelenkpfanne ermöglicht wird.

Vorteilhafterweise sind beliebige Gelenkpfannen mit der erfindungsgemäß ausgestalteten Femurkomponente kombinierbar.

Ausführungsbeispiele der Erfindung werden im folgenden anhand teilweise schematischer Darstellungen in der Zeichnung näher erläutert. In der Zeichnung zeigen:

Fig. 1 eine schematische Ansicht eines ersten Ausführungsbeispiels einer erfindungsgemäß ausgestalteten Femurkomponente einer Hüftgelenks-Endoprothese,

Fig. 2A-D verschiedene Querschnittsformen der Halspartie einer Femurkomponente sowohl gemäß dem Stand der Technik als auch gemäß der Erfindung entlang der Schnittebene II-II in Fig. 1,

Fig. 3A-B erläuternde Darstellungen einer Hüftgelenks-Endoprothese für den technischen Range of motion,

Fig. 4A-B erläuternde Darstellungen einer Hüftgelenks-Endoprothese für den anatomischen Range of motion,

Fig. 5A-D schematische Darstellungen der Berührungspunkte der einzelnen Komponenten der Hüftgelenks-Endoprothese für verschiedene Querschnittsformen,

Fig. 6A-B schematische Darstellungen der Krümmungsradien zweier verschiedener ovaler Querschnittsformen des Anschlagbereiches, und

Fig. 7 ein zweites Ausführungsbeispiel einer erfindungsgemäß ausgestalteten Femurkomponente.

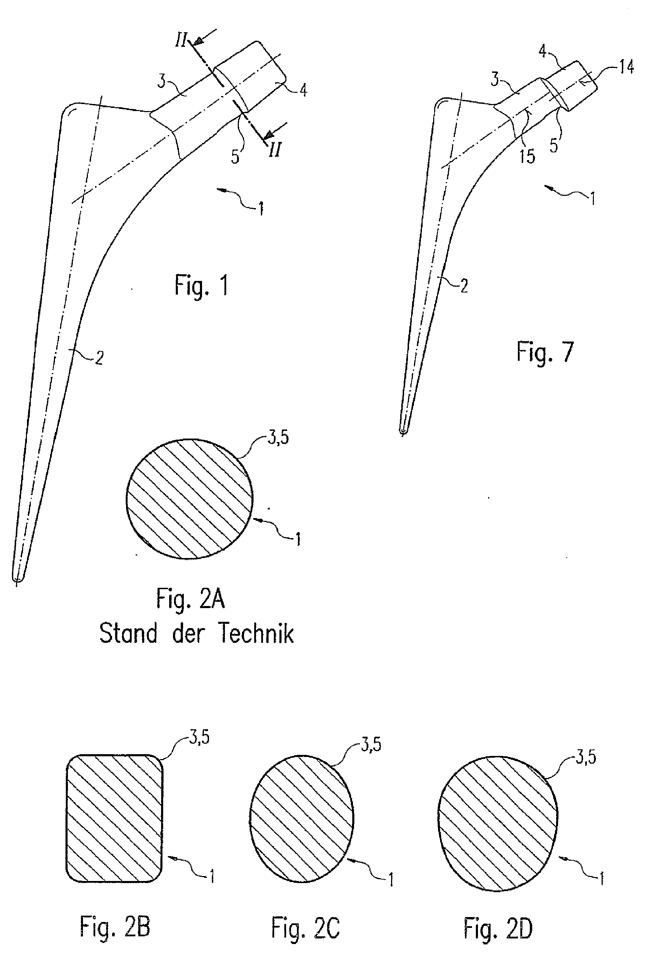

Fig. 1 zeigt in einer schematischen Darstellung eine Ansicht einer Femurkomponente 1 für eine totale Hüftgelenks-Endoprothese. Die Femurkomponente 1 weist einen Schaft 2 auf, welcher in der Markhöhle eines Femurknochens je nach Bauform der Femurkomponente 1 einzementiert oder zementfrei verankert wird. An dem Schaft 2 ist eine Halspartie 3 ausgebildet, an deren Ende ein Zapfen 4 zur Aufnahme eines Gelenkkopfes 8 ausgebildet ist. Der Gelenkkopf 8 sowie die entsprechende Gelenkpfanne 6 sind in Fig. 1 nicht weiter dargestellt.

Fig. 2A bis 2D zeigen in einer schematischen geschnittenen Darstellung mögliche Querschnittsformen für den Anschlagsbereich 5 zwischen dem Zapfen 4 und dem Schaft 2 der Femurkomponente 1.

In Fig. 2A ist dabei eine Querschnittsform gemäß dem Stand der Technik dargestellt. Hierbei handelt es sich um eine runde Querschnittsform, welche in jeder Bewegungsrichtung einen identischen Range of motion erlaubt, welcher lediglich durch die Form der verwendeten Gelenkspfanne limitiert ist.

Die Fig. 2B bis 2D zeigen abgewandelte Querschnittsformen, die beispielsweise rechteckig, elliptisch oder oval geformt sein können. Bedingt durch die weiter unten näher erläuterte Definition eines sogenannten Range of Motion bieten solche nicht kreisrunden Querschnittsformen für bestimmte Bewegungsabläufe eine größere Bewegungsfreiheit.

Die in den Ausführungsbeispielen dargestellte Hüftgelenks-Endoprothese wird auch als totale Hüftgelenks-Endoprothese bezeichnet, da sowohl der femurale als auch der beckenseitige Gelenkanteil ersetzt wird. Eine solche Hüftgelenks-Endoprothese weist eine definierte Beweglichkeitsspielraum auf, welcher als Range of motion bezeichnet wird. Dieser ist durch die Querschnittsform der Halspartie 3 der Femurkomponente 1 sowie die Form der im Becken verankerten Gelenkpfanne 6 bestimmt.

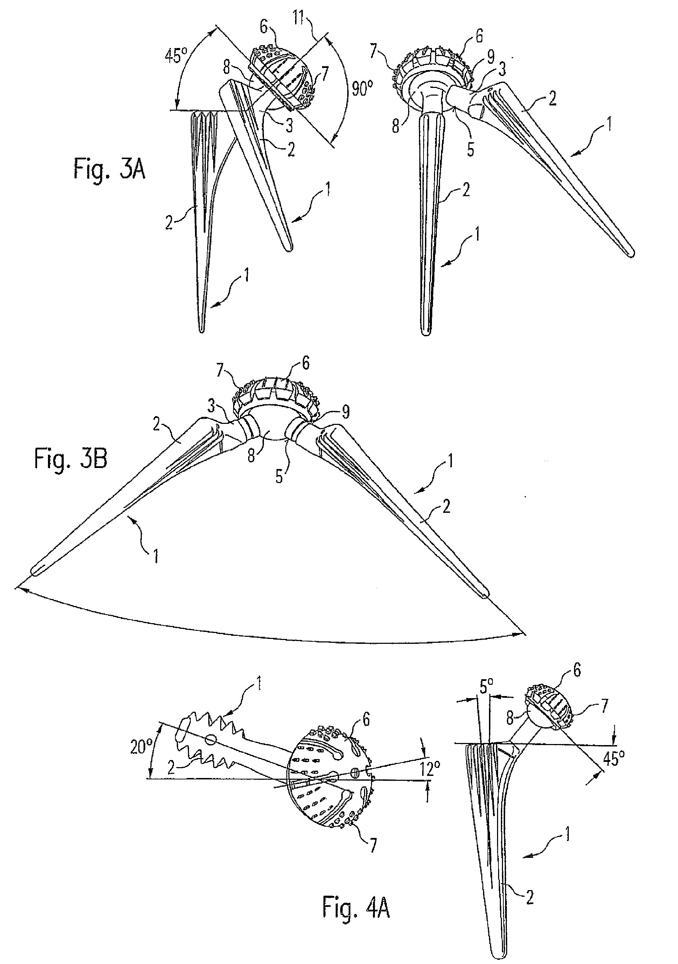

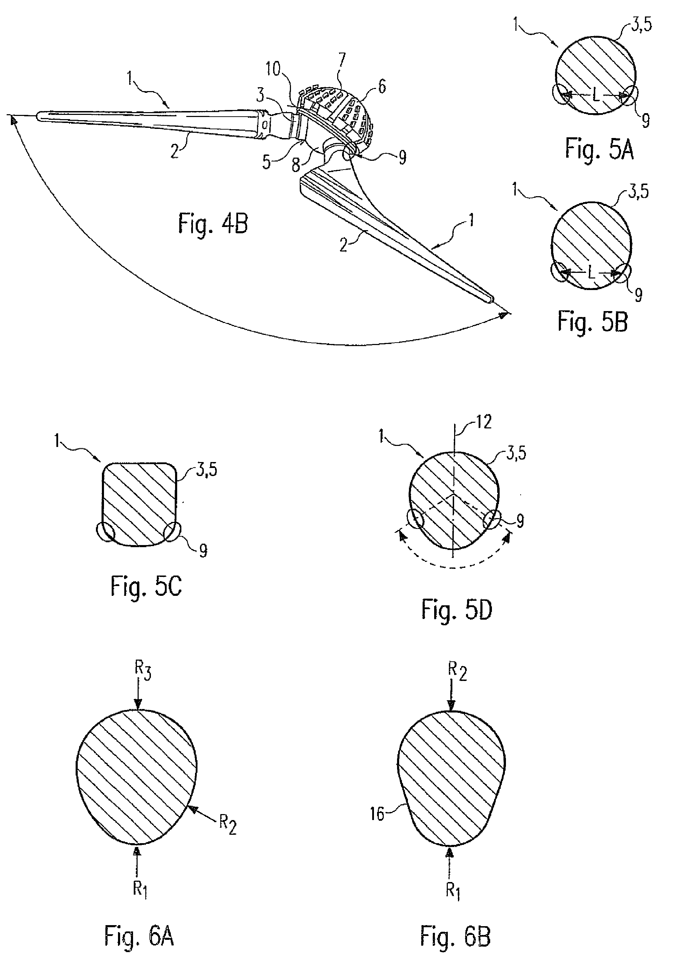

Fig. 3A und 3B sowie Fig. 4A und 4B zeigen den technischen und den anatomischen Range of motion anhand schematischer Darstellungen einer Hüftgelenks-Endoprothese mit einer Femurkomponente 1 und einer entsprechenden Gelenkpfanne 6 zur Implantation in den Beckenknochen. In dem in den vorliegenden Figuren beschriebenen Ausführungsbeispiel handelt es sich um eine Spreizpfanne 6 mit Widerhaken 7, welche zementlos in das Gewebe des Beckenknochens eingesetzt wird und über einen an dem Zapfen 4 an der Halspartie 3 ausgebildeten Kugelkopf 8 mit der Femurkomponente 1 zusammenwirkt. Die Spreizpanne 6 umschließt dabei den Kugelkopf 8 so weit, daß eine Passung erzielt wird, welche den Kugelkopf 8 in der Spreizpfanne 6 hält.

Die im folgenden näher beschriebenen erfindungsgemäßen Maßnahmen sind unabhängig von der Form der Spreizpfanne 6 zu sehen. Um den Range of motion für verschiedene Querschnittsformen des Anschlagsbereiches 5 zu vergleichen, müssen lediglich jeweils gleiche Hüftgelenks-Endoprothesen mit gleichen Komponenten, also beispielsweise mit gleichen Gelenkpfannen 6 miteinander verglichen werden, die Form der Gelenkpfanne 6 ist dabei jedoch nicht auf die dargestellte Spreizpfanne 6 beschränkt, sondern kann auch in Form einer den Gelenkkopf 8 nicht kraftschlüssig umfassenden Gelenkpfanne 6 ausgebildet sein. Die verschiedenen Gelenkpfannen 6 haben unterschiedliche Vor- und Nachteile. Einerseits ist der Range of motion bei einer den Gelenkkopf umgreifenden Spreizpfanne 6 geringer, dafür ist jedoch auch die Luxationsgefahr geringer. Andererseits ist bei einer Gelenkpfanne 6, welche den Gelenkkopf 8 weniger stark umgreift, die Gefahr einer Luxation größer, jedoch ist auch der Range of motion größer.

In den Fig. 3A und 3B ist der maximale technische Range of motion für eine Bewegung zwischen zwei Positionen maximaler Auslenkung der Femurkomponente 1 in der Gelenkpfanne 6 dargestellt. Der maximale technische Range of motion ist dabei durch den physikalisch maximal möglichen Winkelbereich zwischen zwei Berührungspunkten 9 jeweils an einem Rand 10 der Spreizpfanne 6 ausgehend von der in Fig. 3A dargestellten Ausgangslage ohne Verkippung der Spreizpfanne 6 relativ zu einer Längsachse 11 des Halsbereiches 3 definiert, wie in Fig. 3B dargestellt.

Der technische Range of motion gemäß dem Stand der Technik für einen runden Querschnitt im Anschlagsbereich 5 der Halspartie 3 und eine Spreizpfanne 6 gemäß den Figuren beträgt für eine definierte Konstellation der Größenverhältnisse der einzelnen Komponenten ca. $110° < \alpha < 130°$.

Der anatomische Range of motion unterscheidet sich von dem technischen Range of motion dadurch, daß er nicht die technisch maximal mögliche Flexion, sondern die anatomisch maximal mögliche aktive und passive Flexion in Flexions-/Extensionsrichtung, also in einer Richtung entlang der Bewegungsabläufe des Beugens und Streckens des Oberschenkels relativ zum Becken, beschreibt. Aktive Flexion tritt im Rahmen der natürliche Bewegung bei Aktivitäten wie beispielsweise Gehen und Sitzen auf, während die passive Flexion durch Zuhilfenahme von anderen Gliedmaßen (Beispiel: Heranziehen des gebeugten Beins Richtung Brust mit den Armen) oder durch eine andere Person (Beispiel: Krankengymnastik mit gezielter Muskeldehnung) ausgeübt wird.

Der anatomische Range of motion gemäß dem Stand der Technik für einen runden Querschnitt im Anschlagsbereich 5 der Halspartie 3 und eine Spreizpfanne 6 gemäß den Figuren liegt für eine definierte geometrische Konstellation der einzelnen Komponenten bei $120° < \alpha < 160°$. Entsprechende Werte sind dabei in Fig. 4A und 4B beispielhaft wiedergegeben. Die Inklination der Spreizpfanne 6 zum Schaft 2 beträgt dabei 45°, die Ausrichtung im Beckenknochen beträgt in Anteversion 12° und in Antetorsion 20°. Der Femurwinkel beträgt 5°.

Im Licht der vorangegangenen Ausführungen wird verständlich, daß ein erweiterter anatomischer Range of motion bei gleichbleibender Stabilität des Gelenks angestrebt wird. Die Biegsteifigkeit und die Festigkeit der Gelenkverbindung darf dabei ebensowenig leiden wie die Stabilität gegenüber Luxationen. Die erfindungsgemäßen Maßnahmen im Anschlagsbereich 5 der Halspartie 3 der Femurkomponente 1 der Hüftgelenks-Endoprothese ermöglichen eine signifikante Erweiterung des anatomischen Range of motion.

Dies wird dadurch erzielt, daß die runde Querschnittsfläche im Anschlagsbereich 5 der Halspartie 3 so verformt wird, daß die Berührungspunkte 9 in ihrer Lage zueinander verschoben werden und dadurch ein größerer Winkelbereich zugänglich gemacht wird.

Die Fig. 5A bis 5D zeigen die unterschiedlichen Querschnittsformen des Anschlagsbereichs 5 der Halspartie 3 gemäß Fig. 2A bis 2D. Zur Verdeutlichung sind die Zonen, in welchen die Berührungspunkte 9 liegen, markiert.

Vergleicht man beispielsweise die Fig. 5A mit dem kreisförmigen Querschnitt gemäß dem Stand der Technik und 5B mit einem elliptischen Querschnitt, ist erkennbar, daß ein linearer Abstand L der Berührungspunkte 9 bei ansonsten gleichbleibender Geometrie kleiner wird und sich dadurch der maximale Winkel vergrößert.

Ein ähnlicher Effekt tritt für einen rechteckigen Querschnitt mit abgerundeten Ecken gemäß Fig. 5C auf. Je stärker die beiden medial liegenden Ecken abgerundet sind, desto größer fällt der anatomische Range of motion aus.

Betrachtet man Fig. 5D mit einem ovalen Querschnitt, fällt auf, daß sich nicht nur der Abstand L der Berührungspunkte 9 verkleinert, sondern daß diese sich vorteilhafterweise auch verschieben, so daß ein Öffnungswinkel zwischen den Berührungspunkten 9 und einer Achse 12 größer wird.

Der anatomische Range of motion vergrößert sich dabei von ca. 152° bei einer runden Querschnittsform gemäß Fig. 5A über ca. 159° bei einer elliptischen und ca. 161° bei einer rechteckigen Querschnittsform gemäß Fig. 5B und 5C zu ca. 165° bei einer ovalen Querschnittsform gemäß Fig. 5D bei ansonsten gleichbleibenden geometrischen Gegebenheiten.

Die Differenz beträgt also in diesem Ausführungsbeispiel bis zu 13° für den vollständigen Öffnungswinkel zwischen den Berührungspunkten 9 bzw. etwa 8%.

In den Fig. 6A und 6B sind schematisch Krümmungsradien für ovale Querschnittsformen des Anschlagbereiches 5, die beispielsweise aus Fig. 2D und 5D hervorgehen, dargestellt.

Betrachtet man Fig. 6A, ist ersichtlich, daß die ovale Querschnittsform durch drei unterschiedliche Krümmungsradien $R_1$, $R_2$ und $R_3$ beschrieben werden kann. Dabei gilt, daß dabei

$$R_1 < R_3 < R_2$$

gilt.

Dabei ist der kleinste Krümmungsradius $R_1$ im medialen Bereich, der mittlere Krümmungsradius $R_3$ im lateralen Bereich und der größte Krümmungsradius $R_2$ im dazwischen liegenden Übergangsbereich vorhanden. Beispielsweise liegt $R_1$ im Bereich von 4 mm bis 5 mm, besonders bevorzugt ca. 4,3 mm, $R_3$ im Bereich von 5 mm bis 8 mm, besonders bevorzugt ca. 5,3 mm und $R_2$ im Bereich von 10 mm bis 20 mm, besonders bevorzugt ca. 15 mm.

Eine einfachere ovale Querschnittsform mit gerade Flanken 16 ist durch nur zwei Krümmungsradien $R_1$ und $R_2$ beschreibbar, für welche gilt:

$$R_1 < R_2.$$

Dabei ist der kleinere Krümmungsradius $R_1$ im medialen Bereich und der größere Krümmungsradius $R_2$ im lateralen Bereich vorhanden. Beispielsweise liegt $R_1$ im Bereich von 4 mm bis 5 mm, besonders bevorzugt ca. 4,3 mm, und $R_2$ im Bereich von 5 mm bis 8 mm, besonders bevorzugt ca. 5,3 mm.

Die weiter oben beschriebene Verkürzung des linearen Abstandes L der Berührungspunkte 9 ist dabei auch auf die in Fig. 6B dargestellte ovale Querschnittsform des Anschlagbereiches 5 übertragbar.

In Fig. 7 ist eine weitere Möglichkeit der Erweiterung des Range of Motion dargestellt. Statt, wie in den vorhergehenden Ausführungsbeispielen, die Querschnittsform der Halspartie 3 im Anschlagsbereich 5 zu modifizieren, kann auch eine Mittelachse 14 des Zapfens 4 zur Aufnahme des Kugelkopfes 8, wie in Fig. 6 dargestellt, exzentrisch zu einer Mittelachse 15 der Halspartie 3 angeordnet sein. Die Richtung der Verschiebung des Zapfens 4 muß dabei entsprechend gewählt werden, sie erfolgt vorzugsweise in medialer Richtung. Dadurch kann ebenfalls der Range of motion erweitert werden, da in dem kritischen Anschlagsbereich 5, wo die Halspartie 3 in Berührung mit dem Rand 10 der Spreizpfanne 6 kommt, eine Kante 13 ausgebildet ist, welche außerhalb des Randes 10 der Spreizpfanne 6 liegt und so eine stärkere Abwinkelung ermöglicht, da sie nicht an dem Rand 10 ansteht, sondern diesen unterschneidet.

Durch eine Verschiebung des Zapfens 4 sowohl in lateraler als auch gleichzeitig in anteriorer Richtung kann der anatomische Range of motion noch gezielter für die Hüftbeugung oder -streckung bei gleichzeitiger Beinrotation optimiert werden.

Die Erfindung ist nicht auf die dargestellten Ausführungsbeispiele beschränkt und auch für andere Formen von Gelenkpfannen 6 und Femurkomponenten 1 anwendbar.

## Patentansprüche

1. Femurkomponente (1) für eine Hüftgelenks-Endoprothese, mit einem in einer Markhöhle eines Femurknoschens verankerbaren Schaft (2) und einer daran ausgebildeten Halsparite (3) mit einem Zapfen (4) zur Aufnahme eines Gelenkkopfes (8), wobei die Halspartie (3) in einem zwischen dem Zapfen (4) und dem Schaft (2) ausgebildeten Anschlagsbereich (5) einen nicht runden Querschnitt aufweist, **dadurch gekennzeichnet,** **dass** bei einer ovalen Ausbildung des Querschnitts des Anschlagsbereiches (5) mit zwei verschiedenen Krümmungsradien ($R_1$, $R_2$) und geraden Flanken (16) gilt:

$$R_1 < R_2,$$

wobei

$R_1$ der Krümmungsradius im medialen Bereich

$R_2$ der Krümmungsradius im lateralen Bereich ist.

**2.** Femurkomponente nach Anspruch 1,
**dadurch gekennzeichnet,**
**dass** der Querschnitt des Anschlagsbereichs (5) oval, im wesentlichen elliptisch oder im wesentlichen rechtekkig ist.

**3.** Femurkomponente nach Anspruch 2,
**dadurch gekennzeichnet,**
**dass** die Halspartie (3) außerhalb des Anschlagsbereichs (5) einen runden Querschnitt aufweist.

**4.** Femurkomponente nach einem der Ansprüche 1 bis 3,
**dadurch gekennzeichnet,**
**dass** die Femurkomponente (1) im Anschlagsbereich (5) Berührungspunkte (9) mit einem Rand (10) einer Gelenkpfanne (6) der Hüftgelenks-Endoprothese aufweist.

**5.** Femurkomponente nach Anspruch 4,
**dadurch gekennzeichnet,**
**dass** durch die nicht runde Ausformung des Anschlagbereiches (5) ein Öffnungswinkel ($\alpha$) zwischen den Berührungspunkten (9) gegenüber einer runden Ausformung vergrößert ist.

**6.** Femurkomponente nach Anspruch 5,
**dadurch gekennzeichnet,**
**dass** durch die nicht runde Ausformung des Anschlagbereiches (5) ein linearer Abstand (L) zwischen den Berührungspunkten (9) verkleinert ist.

**7.** Femurkomponente nach einem der Ansprüche 1 bis 6,
**dadurch gekennzeichnet,**
**dass** auf den Zapfen (4) ein mit der Gelenkpfanne (6) zusammenwirkende Gelenkkopf (8) aufsteckbar ist.

Fig. 1

Fig. 2A
Stand der Technik

Fig. 7

Fig. 2B

Fig. 2C

Fig. 2D

Fig. 3A

Fig. 3B

Fig. 4A

Fig. 4B

Fig. 5A

Fig. 5B

Fig. 5C

Fig. 5D

Fig. 6A

Fig. 6B

Europäisches
Patentamt

European
Patent Office

Office européen
des brevets

**EUROPÄISCHER RECHERCHENBERICHT**

**Nummer der Anmeldung**

EP 12 16 9546

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 0 865 777 A (BIOMET MERCK FRANCE; ORA) 23. September 1998 (1998-09-23) * Abbildungen 4,6,7 * * Spalte 5, Zeile 18 - Zeile 20 * ----- | 1-7 | INV. A61F2/36 A61F2/32 |
| A | FR 2 824 259 A (GROUPE LEPINE) 8. November 2002 (2002-11-08) * Abbildungen 1,3,4 * * Seite 3, Zeile 19 - Zeile 26 * ----- | 1-7 | |
| A | US 5 387 244 A (BREARD ET AL) 7. Februar 1995 (1995-02-07) * Abbildungen 1,4,5 * * Spalte 1, Zeile 27 - Zeile 30 * * Spalte 3, Zeile 9 - Zeile 14 * ----- | 1-7 | |
| A | US 2002/038148 A1 (FERNANDEZ JOSE ET AL) 28. März 2002 (2002-03-28) * Abbildung 1 * * Absatz [0019] * ----- | 1-7 | RECHERCHIERTE SACHGEBIETE (IPC) A61F |
| A | EP 0 797 964 A (GROUPE LEPINE) 1. Oktober 1997 (1997-10-01) * Abbildungen 1,10,11 * ----- | 1-7 | |

Der vorliegende Recherchenbericht wurde für alle Patentansprüche erstellt

| Recherchenort | Abschlußdatum der Recherche | Prüfer |
|---|---|---|
| München | 7. August 2012 | Josten, Stefan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 12 16 9546

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-08-2012

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| EP 0865777 | A | | 23-09-1998 | DE | 69808163 | D1 | 31-10-2002 |
| | | | | DE | 69808163 | T2 | 06-02-2003 |
| | | | | EP | 0865777 | A1 | 23-09-1998 |
| | | | | ES | 2182249 | T3 | 01-03-2003 |
| | | | | FR | 2760966 | A1 | 25-09-1998 |
| FR 2824259 | A | | 08-11-2002 | | | | |
| US 5387244 | A | | 07-02-1995 | FR | 2638350 | A1 | 04-05-1990 |
| | | | | US | 5387244 | A | 07-02-1995 |
| US 2002038148 | A1 | | 28-03-2002 | AT | 298547 | T | 15-07-2005 |
| | | | | AU | 771968 | B2 | 08-04-2004 |
| | | | | AU | 4567701 | A | 24-09-2001 |
| | | | | CA | 2374072 | A1 | 20-09-2001 |
| | | | | CN | 1366457 | A | 28-08-2002 |
| | | | | DE | 60111703 | D1 | 04-08-2005 |
| | | | | DE | 60111703 | T2 | 08-12-2005 |
| | | | | EP | 1191906 | A1 | 03-04-2002 |
| | | | | ES | 2240435 | T3 | 16-10-2005 |
| | | | | HK | 1048584 | A1 | 27-01-2006 |
| | | | | JP | 3834510 | B2 | 18-10-2006 |
| | | | | JP | 2003526454 | A | 09-09-2003 |
| | | | | PT | 1191906 | E | 31-10-2005 |
| | | | | TW | 487568 | B | 21-05-2002 |
| | | | | US | 6319286 | B1 | 20-11-2001 |
| | | | | US | 2002038148 | A1 | 28-03-2002 |
| | | | | WO | 0167997 | A1 | 20-09-2001 |
| EP 0797964 | A | | 01-10-1997 | EP | 0797964 | A1 | 01-10-1997 |
| | | | | FR | 2746631 | A1 | 03-10-1997 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9823231 A1
- WO 8302555 A1
- EP 0048745 A1
- EP 0093378 A1